# EUROPEAN PATENT APPLICATION

(11) **EP 4 381 945 A1**
(43) Date of publication of application: **12.06.2024**
(21) Application number: 22852976.4
(22) Date of filing: 29.07.2022
(51) Int. Cl.: A01N 59/00, A61L 2/02, A61L 2/232

(54) **ANTIBACTERIAL METAL MATERIAL AND ANTIBACTERIAL ARTICLE**

(30) Priority: 05.08.2021 JP 2021129074
(71) Applicant: Mitsui Chemicals, Inc., Tokyo 104-0028 (JP)
(72) Inventor: MITSUNAGA, Shintaro, Sodegaura-shi, Chiba 299-0265 (JP)
(74) Representative: J A Kemp LLP
(86) International application number: PCT/JP2022/029360
(87) International publication number: WO 2023/013557

(57) **Abstract**

Provided is an antibacterial metal material that includes a surface having a roughness index of 4.0 or more. The roughness index is determined by dividing a true surface area (m²), which is measured by a krypton adsorption method, by a geometric surface area (m²).

## Description

### Technical Field

The present disclosure relates to an antibacterial metal material and an antibacterial article.

### Background Art

There is an increasing demand for antibacterial articles having an antibacterial property in many applications, including pharmaceutical/medical-related applications and housing-related applications. For example, Patent Document 1 discloses an antibacterial article provided with an antibacterial resin film. The antibacterial article disclosed in Patent Document 1 includes an antibacterial resin film containing a specific polymer, and a substrate to which the antibacterial resin film is bonded.
Patent Document 1: WO 2016/051444

### SUMMARY OF THE INVENTION

### Technical Problem

In the technology disclosed in Patent Document 1, the antibacterial resin film is potentially peeled off from the substrate, and elution of the chemical substances contained in the antibacterial resin film is a concern. The antibacterial property of the antibacterial resin film may not be sufficient.

The disclosure was made in view of the above-described circumstances, and an object of one embodiment of the disclosure is to provide an antibacterial metal material having excellent antibacterial property.

An object of another embodiment of the disclosure is to provide an antibacterial article using the antibacterial metal material.

### Solution to Problem

The disclosure encompasses the following aspects.
<1> An antibacterial metal material, including a surface having a roughness index of 4.0 or more as determined by dividing a true surface area (m²), which is measured by a krypton adsorption method, by a geometric surface area (m²).
<2> The antibacterial metal material according to <1>, wherein the surface has a porous structure.
<3> The antibacterial metal material according to <2>, including a plating layer, wherein the surface is a surface of the plating layer.
<4> The antibacterial metal material according to <2> or <3>, including a structure in which at least some respective pores among a pluraity ofl pores have an opening diameter that is smaller than a maximum inner diameter of an interior of the respective pores.
<5> The antibacterial metal material according to any one of <2> to <4>, wherein the surface satisfies at least one of the following (1) to (3):
   (1) an average value of an arithmetic average roughness (Ra) is from 0.2 µm to 20 µm;
   (2) an average value of a ten-point average roughness (Rz) is from 2 µm to 100 µm; and
   (3) an average value of an average length (RSₘ) of profile elements is from 10 µm to 400 µm.
<6> The antibacterial metal material according to <5>, wherein
   the average value of the arithmetic average roughness (Ra) is 3 µm or more,
   the average value of the ten-point average roughness (Rz) is 20 µm or more, and
   the average value of the average length (RSₘ) of profile elements is 100 µm or more.
<7> The antibacterial metal material according to any one of <1> to <6>, wherein the surface includes an uneven structure layer having an average thickness of from 10 nm to 5,000 nm.
<8> The antibacterial metal material according to <7>, wherein the uneven structure layer is a dendritic layer or a mesh-like layer.
<9> The antibacterial metal material according to <7> or <8>, wherein the average thickness is more than 200 nm.
<10> The antibacterial metal material according to <7> or <8>, wherein the average thickness is 350 nm or more.
<11> The antibacterial metal material according to any one of <1> to <10>, containing at least one selected from the group consisting of aluminum, magnesium, silver, copper, iron, titanium, an alloy, and a plating material,
   wherein the alloy contains at least one selected from the group consisting of aluminum, magnesium, silver, copper, iron, and titanium.
<12> The antibacterial metal material according to any one of <1> to <11>, wherein the roughness index is 95.0 or more.
<13> An antibacterial article, including the antibacterial metal material according to any one of <1> to <12>.

### Advantageous Effects of Invention

According to one embodiment of the disclosure, an antibacterial metal material having excellent antibacterial property is provided.

According to another embodiment of the disclosure, an antibacterial article using the antibacterial metal material is provided.

### BRIEF DESCRIPTION OF DRAWING

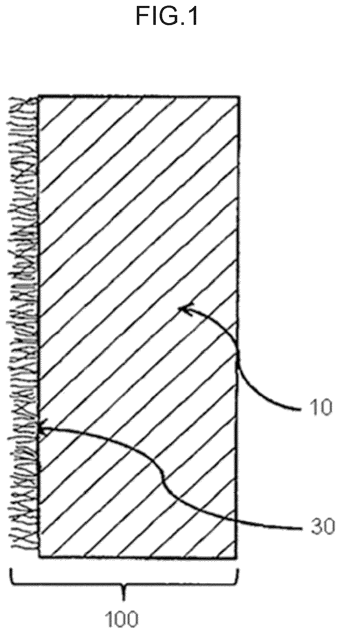

FIG. 1 is a cross-sectional view schematically illustrating one example of a dendritic layer of an antibacterial metal material.

### DESCRIPTION OF EMBODIMENTS

In the disclosure, those numerical ranges that are expressed with "to" each means a range that includes the numerical values stated before and after "to" as the minimum value and the maximum value, respectively.

In a set of numerical ranges that are stated in a stepwise manner in the disclosure, the upper limit value or the lower limit value of one numerical range may be replaced with the upper limit value or the lower limit value of other numerical range, or may be replaced with a relevant value indicated in any of Examples.

In the disclosure, when there are plural kinds of substances that correspond to a component, an indicated amount of the component means, unless otherwise specified, a total amount of the plural kinds of substances.

In the disclosure, the term "step" encompasses not only a discrete step but also a step that cannot be clearly distinguished from other steps, as long as the intended purpose of the step is achieved.

In the disclosure, where an embodiment is described referring to the drawing, the constitution of the embodiment is not limited to the one illustrated in the drawing. The sizes of the members in the drawing are conceptual, and the relative size relationships among the members are not limited to those illustrated in the drawing.

### <Antibacterial Metal Material>

The antibacterial metal material of the disclosure includes a surface having a roughness index of 4.0 or more as determined by dividing a true surface area (m²), which is measured by a krypton adsorption method, by a geometric surface area (m²). This allows the antibacterial metal material to exhibit excellent antibacterial property.

In the description of the antibacterial metal material of the disclosure, the term "antibacterial property" means at least one of antibacterial performance or virus inactivation performance. The term "bacteria" used herein is a concept that encompasses bacteria and virus.

The bacterial against which the antibacterial metal material of the disclosure exhibits its antibacterial performance include *Staphylococcus aureus.* The virus against which the antibacterial metal material of the disclosure exhibits its virus inactivation performance include influenza A H3N2 and feline calicivirus. Hereinafter, according to the below-described presumed mechanism, bacteria species against which the antibacterial performance is exhibited are not limited to *Staphylococcus aureus,* and virus species against which the virus inactivation performance is exhibited are not limited to influenza A H3N2 and feline calicivirus.

The antibacterial metal material of the disclosure encompasses a first configuration and a second configuration. The first configuration consists of only the below-described member formed of a metal or an alloy. The second configuration includes the member formed of a metal or an alloy, and a plating layer disposed on the member. In the case of the first configuration, a "surface" of the antibacterial metal material refers to a surface of the member formed of a metal or an alloy. In the case of the second configuration, a "surface" of the antibacterial metal material refers to a surface of the plating layer.

The mechanism in which the antibacterial metal material is allowed to exhibit excellent antibacterial property by controlling the surface roughness index (hereinafter, also simply referred to as "roughness index") of the antibacterial metal material to be 4.0 or more is not clear; however, it is presumed as follows.

A surface of the antibacterial metal material, which has a roughness index of 4.0 or more, has a fine uneven structure. Therefore, when bacteria adhere to this surface (i.e., uneven structure) of the antibacterial metal material, it is believed that the uneven structure damages the bacteria, or kills the bacteria in a preferred aspect.

When viruses adhere to the uneven structure of the antibacterial metal material, it is believed that at least some of the viruses are captured by the uneven structure, as a result of which the activities of the viruses are weakened, or the viruses are inactivated in a preferred aspect.

The scope of the disclosure is not limited by the above-described presumption.

As described below in detail, the antibacterial metal material can be produced by performing a roughening treatment on a surface of a metal material, and a fine uneven structure is formed on the surface of the metal material by the roughening treatment. In other words, by roughening the surface of the metal material itself, an uneven structure having an antibacterial property is formed. Accordingly, such peeling as described above for the technology disclosed in Patent Document 1 using an antibacterial resin film does not occur, so that elution of chemical substances is inhibited. In addition, in an aspect in which the antibacterial property of a metal itself is exerted, the antibacterial property of the antibacterial metal material can be further improved.

From the standpoint of further improving the antibacterial property, the roughness index is preferably 10.0 or more, more preferably 25.0 or more, still more preferably 50.0 or more, particularly preferably 95.0 or more. When the roughness index is 95.0 or more, not only superior antibacterial performance against *Staphylococcus aureus* and superior virus inactivation performance against influenza A H3N2 are obtained, but also *Staphylococcus aureus* can be further damaged and the activities of influenza A H3N2 and feline calicivirus can be weakened in a relatively short time (e.g., after a lapse of 30 minutes from the inoculation of the bacteria or virus). As a result, the antibacterial metal material exhibits superior antibacterial property as well as excellent immediate effect.

An upper limit value of the roughness index is not particularly limited; however, from the standpoint of further improving the antibacterial property, the roughness index may be 150.0 or less, 125.0 or less, or 110.0 or less.

In the disclosure, the true surface area is a value calculated by multiplying the specific surface area (m²/g) of a sample (i.e., antibacterial metal material) obtained by a krypton adsorption method by the mass of the sample.

The specific surface area is determined by the BET method after vacuum-heating and degassing (100°C) the sample (antibacterial metal material) and subsequently measuring the adsorption isotherm by a krypton adsorption method under the liquid nitrogen temperature (77 K). The adsorption isotherm can be measured using a gas adsorption amount measuring device. As the gas adsorption amount measuring device, for example, BELSORP-MAX (manufactured by MicrotracBEL Corp.) may be employed.

As the mass of the antibacterial metal material, the mass of the metal material after the roughening treatment may be used. Since the change in the mass before and after the roughening treatment is very small, the mass of the metal material prior to the roughening treatment may also be used as the mass of the antibacterial metal material.

In the disclosure, the geometric surface area is a value determined from the dimensions of a measurement object. For example, when the measurement object is a rectangular parallelepiped of X in length, Y in width, and Z in height, the geometric surface area S is calculated by: S = 2XY + 2YZ + 2ZX.

The measurement object of the geometric surface area is the surface of a part or the entirety of the antibacterial metal material.

The measurement object of the geometric surface area may be the metal material subjected to the roughening treatment. Since the change in the geometric surface area before and after the roughening treatment is very small, the measurement object of the geometric surface area may also be the metal material prior to the roughening treatment.

The type of a metal contained in the antibacterial metal material is not particularly limited. Examples of the metal include aluminum, iron, copper, nickel, gold, silver, platinum, cobalt, zinc, lead, tin, zirconium, titanium, niobium, chromium, aluminum, magnesium, manganese, and alloys containing the above-described metals, as well as plating materials of the above-described metals and alloys.

For example, the antibacterial metal material may contain at least one selected from the group consisting of aluminum, magnesium, silver, copper, iron, titanium, an alloy, and a plating material. The alloy contains at least one selected from the group consisting of aluminum, magnesium, silver, copper, iron, and titanium. The alloy may contain aluminum, magnesium, silver, copper, iron, and titanium as a main component. The term "main component" used herein indicates that the ratio of aluminum, magnesium, silver, copper, iron, and titanium is 50% by mass or more with respect to a total amount of the alloy. The plating material contains aluminum, magnesium, copper, iron, titanium, and the above-described alloy.

Particularly, silver and copper can, because of their antibacterial properties, further improve the antibacterial property of the antibacterial metal material.

Examples of the alloy include yellow copper, phosphor bronze, steel (e.g., stainless steel), and brass. Examples of the plating material include plated steel.

A surface of the antibacterial metal material may have a porous structure (hereinafter, may be referred to as "first fine structure").

The term "porous structure" used herein refers to a structure having plural pores. Specifically, the term "porous structure" refers to a structure in which pores are present when a cross-section obtained by cutting the antibacterial metal material horizontally with respect to a surface of the antibacterial metal material is observed.

The term "pore" used herein means an open pore (a pore connected to the open air). The size of a pore is a value measured at the inlet of the pore.

The porous structure can be verified by observing a surface of the antibacterial metal material and a cross-section perpendicular to the surface under an electron microscope or a laser microscope.

It is preferred that the antibacterial metal material has a plating layer, and that a surface of the porous structure is a surface of the plating layer. Examples of a material of the plating layer include zinc, nickel, and chromium.

Hereinafter, a plating layer whose surface has a porous structure may be referred to as "porous plating layer".

The antibacterial metal material may have a porous structure in which at least some respective pores among a plurality of pores have an opening diameter that is smaller than a maximum inner diameter of an interior of the respective pores (hereinafter, this structure may be referred to as "specific structure"). This can make it easier to improve the antibacterial property of the antibacterial metal material.

In the disclosure, the "opening diameter" of a pore is a value measured at the inlet of the pore.

The specific structure can be verified by observing a cross-section perpendicular to a surface of the antibacterial metal material under an electron microscope or a laser microscope.

A surface of the antibacterial metal material may satisfy at least one of the following (1) to (3), in addition to having a porous structure:
(1) an average value of an arithmetic average roughness (Ra) is from 0.2 µm to 20 µm;
(2) an average value of a ten-point average roughness (Rz) is from 2 µm to 100 µm; and
(3) an average value of an average length (RSₘ) of profile elements is from 10 µm to 400 µm.

By imparting such a roughened surface to the antibacterial metal material, it can be made easier to improve the antibacterial property of the antibacterial metal material.

Hereinafter, the average value of the arithmetic average roughness (Ra), the average value of the ten-point average roughness (Rz), and the average value of the average length (RSₘ) of profile elements may be collectively referred to as "macroscopic surface properties".

The arithmetic average roughness (Ra) is a value determined in accordance with JIS B0601:2001 (corresponding international standard: ISO4287).

The average value of the arithmetic average roughness (Ra) may be from 0.2 µm to 10 µm, from 0.3 µm to 7 µm, or from 3 µm to 6 µm.

The ten-point average roughness (Rz) is a value determined in accordance with JIS B0601:2001 (corresponding international standard: ISO4287).

The average value of the ten-point average roughness (Rz) may be from 5 µm to 100 µm, from 6 µm to 80 µm, from 8 µm to 50 µm, or from 20 µm to 35 µm.

The average length (RSₘ) of profile elements is a value determined in accordance with JIS B0601:2001 (corresponding international standard: ISO4287).

The average value of the average length (RSₘ) of profile elements may be from 50 µm to 350 µm, from 80 µm to 250 µm, from 90 µm to 150 µm, or from 100 µm to 130 µm.

Particularly, on a surface of the antibacterial metal material, it is preferred that the average value of the arithmetic average roughness (Ra) is 3 µm or more, the average value of the ten-point average roughness (Rz) is 20 µm or more, and the average value of the average length (RSₘ) of profile elements is 100 µm or more. This allows the antibacterial metal material to not only exhibit superior antibacterial performance against *Staphylococcus aureus* and superior virus inactivation performance against influenza A H3N2, but also damage *Staphylococcus aureus* and weaken the activities of influenza A H3N2 and feline calicivirus in a relatively short time (e.g., after a lapse of 30 minutes from the inoculation of the bacteria or virus). As a result, the antibacterial metal material exhibits superior antibacterial property as well as excellent immediate effect.

A surface of the antibacterial metal material may have an uneven structure layer (hereinafter, may be referred to as "second fine structure") having an average thickness of from 10 nm to 5,000 nm. By imparting such a roughened surface to the antibacterial metal material, it can be made easier to improve the antibacterial property of the antibacterial metal material.

The average thickness of the second fine structure may be 20 nm or more but less than 4,000 nm, from 30 nm to 2,000 nm, from 50 nm to 800 nm, from 100 nm to 700 nm, from 150 nm to 600 nm, more than 200 nm but 600 nm or less, or from 350 nm to 600 nm.

When the average thickness of the second fine structure is more than 200 nm, the antibacterial metal material can not only exhibit superior antibacterial performance against *Staphylococcus aureus* and superior virus inactivation performance against influenza A H3N2, but also damage *Staphylococcus aureus* and weaken the activities of influenza A H3N2 and feline calicivirus in a relatively short time (e.g., after a lapse of 30 minutes from the inoculation of the bacteria or virus). As a result, the antibacterial metal material exhibits superior antibacterial property as well as excellent immediate effect.

When the average thickness of the second fine structure is 350 nm or more, the antibacterial metal material exhibits superior antibacterial performance against *Staphylococcus aureus* and superior immediate effect as compared to a case where the average thickness of the second fine structure is more than 200 nm but less than 350 nm.

The average thickness of the second fine structure is calculated from the cross-sectional profile obtained using a scanning electron microscope (SEM). Specifically, using a scanning electron microscope (SEM), SEM images are taken for randomly selected 10 points on the antibacterial metal material, after which the average thickness in a length of 1 µm is measured for 2 arbitrary spots in each image, and the same measurement is performed for other 9 points. An average value of a total of 20 measured points is defined as the average thickness of the second fine structure.

The second fine structure (i.e., uneven structure layer) may be a dendritic layer, a mesh-like layer (hereinafter, may be referred to as "sponge-like layer"), or a pinholder-like layer. This makes it easier to improve the antibacterial property of the antibacterial metal material.

The dendritic layer refers to a layer in which trunks derived from a roughened part of the metal material stand together in large numbers from a surface of the metal material. The trunks may have branches coming out of the respective trunks, and the branches may have side branches coming out of the respective branches.

The mesh-like layer refers to a layer that has a mesh-like or sponge-like shape derived from a roughened part of the metal material.

The pinholder-like layer is a layer that has sharp and randomly-shaped irregularities derived from a plated part subjected to a roughening treatment.

The dendritic layer or the mesh-like layer may be an aluminum oxide layer or an aluminum hydroxide layer. From the standpoint of extending the life of surface irregularities of the metal member in a high-temperature high-humidity environment, the second fine structure preferably includes a dendritic layer made of aluminum oxide.

FIG. 1 illustrates an antibacterial metal material 100 that is one example of the antibacterial metal material of the disclosure.

In the antibacterial metal material 100, for example, as illustrated in FIG. 1, a dendritic layer 30 may be formed on a surface of a metal material 10.

A dendritic layer and a mesh-like layer can be verified by observing a surface of the antibacterial metal material and a cross-section perpendicular to the surface under an electron microscope or a laser microscope.

The above-described configuration allows the antibacterial metal material to have various structures. For example, a surface of the antibacterial metal material may have only a porous structure (first fine structure), only an uneven structure layer (second fine structure), or both of the first fine structure and the second fine structure.

For example, a surface of the antibacterial metal material may have a porous structure and, on this porous structure (first fine structure), a fine uneven structure layer (second fine structure) may be further provided on at least either the inside or the outside of pores. This can make it easy to further improve the antibacterial property of the antibacterial metal material.

For example, a surface of the antibacterial metal material may have a porous structure, and a dendritic layer (second fine structure) may be further formed on at least either the inside or the outside of pores. In this case, the antibacterial metal material may include, for example: a porous structure having at least one of Ra that is from 4 µm to 6 µm, Rz that is from 20 µm to 35 µm, or RSₘ that is from 90 µm to 120 µm; and an uneven structure layer having an average thickness of from several nanometers to several hundred nanometers. This can make it easy to further improve the antibacterial property of the antibacterial metal material.

It is preferred that the presence or absence of the porous structure (first fine structure), the presence or absence of the specific structure, the range of Ra, the range of Rz, the range of RSₘ, the presence or absence and the thickness of the second fine structure, and the presence or absence of the dendritic layer are selected as appropriate in accordance with the types of bacteria and virus against which the antibacterial property is to be exerted. For example, bacteria generally have a size of from several hundred nanometers to several ten micrometers, while virus generally have a size of from several nanometers to several hundred nanometers. Taking into consideration these sizes as well, it is preferred to control the surface structure of the antibacterial metal material (i.e., the above-described features such as the presence or absence of the porous structure, the ranges of Ra and the like, and the presence or absence of the second fine structure) such that the antibacterial property of the antibacterial metal material is exerted in a more favorable manner. For example, for inactivation of viruses having a small size, the antibacterial metal material having a somewhat thick second fine structure can be more effective.

### <Method of Producing Antibacterial Metal Material>

The antibacterial metal material can be produced by performing a roughening treatment on a surface of a metal material. A roughening treatment method is not particularly limited as long as the roughness index of the antibacterial metal material can be controlled at 4.0 or more.

Examples of the roughening treatment method include: a method using a laser as disclosed in Japanese Patent No. 4020957; a method of immersing the surface of the metal material in an aqueous solution of an inorganic base such as NaOH, or an inorganic acid such as HCl or HNO₃; a method of treating the surface of the metal material by anodization as disclosed in Japanese Patent No. 4541153; a substitution-crystallization method in which the surface of the metal material is etched with an aqueous solution containing an acid-based etchant (preferably an inorganic acid, ferric ion, or cupric ion) and, if necessary, manganese ion, aluminum chloride hexahydrate, sodium chloride, or the like, as disclosed in WO 2015/8847 (this method may be hereinafter referred to as "roughening treatment method 1"); a method of immersing the surface of the metal material in an aqueous solution of at least one selected from hydrazine hydrate, ammonia, and a water-soluble amine compound, as disclosed in WO 2009/31632 (this method may be hereinafter referred to as "roughening treatment method 2"); a method of chemically roughening the surface of the metal material through a contact with an oxidizing acidic aqueous solution containing a prescribed metal cation as disclosed in WO 2020/158820 (this method may be hereinafter referred to as "roughening treatment method 3"); a hot water treatment method, such as the one disclosed in Japanese Patent Application Laid-Open (JP-A) No. 2008-162115, 2019-018547, or the like; and a blast treatment. The roughening treatment method can be selected in accordance with a material of the metal material, the desired roughness index, the desired average pore size, and the like.

Among the above-exemplified roughening treatment methods, from the standpoint of controlling the roughness index of the surface of the metal material to be 4.0 or more, the roughening treatment methods 1 to 3 can be preferably employed. In addition, a combination of two or more of these roughening treatment methods (e.g., a roughening treatment method combining the roughening treatment method 1 and roughening treatment method 3) can be preferably employed as well. These treatment methods will now be described concretely.

### [Roughening Treatment Method 1]

One example of the roughening treatment method 1 is a method of performing the following steps (1) to (4) in this order.

### (1) Pre-treatment Step

In the step (1), a pretreatment is performed for removing any oxide film and coating film formed of a hydroxide or the like from a surface of the metal material. As the pretreatment, usually, mechanical polishing or chemical polishing is performed. When the surface of the metal material is significantly contaminated with machine oil and the like, a treatment with an alkaline aqueous solution, such as an aqueous sodium hydroxide solution or an aqueous potassium hydroxide solution, or degreasing may be performed as well.

### (2) Treatment Step with Zinc Ion-Containing Alkaline Aqueous Solution

In the step (2), the metal material subjected to the pretreatment is immersed in a zinc ion-containing alkaline aqueous solution containing an alkali hydroxide (MOH or M(OH)₂) and zinc ion (Zn²⁺) at a mass ratio (MOH or M(OH)₂/Zn²⁺) of from 1 to 100 so as to form a zinc-containing coating film on the surface. It is noted here that M in the above-described MOH and M(OH)₂ represents an alkali metal or an alkaline earth metal.

### (3) Treatment Step with Acid-Based Etchant

In the step (3), after the above-described step (2) is performed, the metal material is treated with an acid-based etchant that contains an acid and at least one of ferric ion or cupric ion. By performing the step (3), not only the zinc-containing coating film on the surface of the metal material can be eluted but also, for example, a porous structure (first fine structure) having at least one of Ra that is from 4 µm to 6 µm, Rz that is from 20 µm to 35 µm, or RSₘ that is from 90 µm to 120 µm can be formed.

### (4) Post-treatment Step

In the step (4), after the above-described step (3) is performed, the metal material is washed. A method of washing the metal material is not particularly limited, and usually consists of washing with water and drying. The method of washing the metal material may include an ultrasonic washing operation for desmutting.

### [Roughening Treatment Method 2]

In the roughening treatment method 2, the metal material is pretreated with hydrochloric acid, sodium hydroxide, nitric acid, or the like, immersed in a weakly basic amine-based aqueous solution of hydrazine hydrate or the like, subsequently washed with water, and then dried at, for example, 70°C or lower. The immersion temperature and the immersion time may be adjusted as appropriate in accordance with the desired roughness index, the desired average pore size, and the like.

As a result, for example, a first fine structure that is a porous structure of several hundred nanometers and a mesh-like second fine structure that is provided on the first fine structure and has an average thickness of several ten nanometers can be formed on a surface of the metal material.

### [Roughening Treatment Method 3]

One example of the roughening treatment method 3 is a method of bringing the metal material into contact with a specific oxidizing acidic aqueous solution. The specific oxidizing acidic aqueous solution contains a metal cation having a standard electrode potential E⁰ at 25°C of -0.2 or higher but 0.8 or lower, preferably higher than 0 but 0.5 or lower.

The oxidizing acidic aqueous solution preferably does not contain any metal cation whose E⁰ is -0.2 or lower.

Examples of the metal cation having a standard electrode potential E⁰ at 25°C of -0.2 or higher but 0.8 or lower include Pb²⁺, Sn²⁺, Ag⁺, Hg²⁺, and Cu²⁺. Thereamong, Cu²⁺ is preferred from the standpoint of the scarcity of the metal as well as the standpoint of the safety and toxicity of corresponding metal salts.

Examples of a compound that generates Cu²⁺ include inorganic compounds, such as copper hydroxide, copper (II) oxide, copper (II) chloride, copper (II) bromide, copper sulfate, copper nitrate, and copper gluconate. Thereamong, copper oxide is preferred from the standpoint of the safety and toxicity as well as the standpoint of the dendritic layer-forming efficiency.

Examples of the oxidizing acidic aqueous solution include nitric acid, a mixed acid, and an aqueous percarboxylic acid solution (e.g., peracetic acid or performic acid). The mixed acid can be obtained by mixing nitric acid with any one of hydrochloric acid, hydrofluoric acid, or sulfuric acid. When nitric acid is used as the oxidizing acidic aqueous solution and copper (II) oxide is used as a metal cation-generating compound, the concentration of nitric acid constituting the aqueous solution is, for example, from 10% by mass to 40% by mass, preferably from 15% by mass to 38% by mass, more preferably from 20% by mass to 35% by mass. The concentration of copper ion constituting the aqueous solution is, for example, from 1% by mass to 15% by mass, preferably from 2% by mass to 12% by mass, more preferably from 2% by mass to 8% by mass.

The temperature at which the metal material is brought into contact with the oxidizing acidic aqueous solution is not particularly limited; however, in order to complete the roughening at an economical speed while managing exothermic reactions, the temperature is, for example, from normal temperature to 60°C, preferably from 30°C to 50°C. In this case, the treatment time is in a range of, for example, from 1 minute to 15 minutes, preferably from 2 minutes to 10 minutes.

In the above-described manner, for example, a dendritic layer (second fine structure) having an average thickness of from several nanometers to several hundred nanometers can be formed on a surface of the metal material by the roughening treatment method 3.

The above-described dendritic layer can also be formed by the hot water treatment method disclosed in JP-A No. 2019-018547.

### [Combination of Roughening Treatment Method 1 and Roughening Treatment Method 3]

In a combination of the roughening treatment method 1 and the roughening treatment method 3, for example, the metal material roughened by the roughening treatment method 1 may be further subjected to the roughening treatment method 3. By this, for example, a porous structure (first fine structure) having at least one of Ra that is from 4 µm to 6 µm, Rz that is from 20 µm to 35 µm, or RSₘ that is from 90 µm to 120 µm, and a dendritic layer (second fine structure) having an average thickness of from several nanometers to several hundred nanometers can be provided on a surface of the metal material.

### <Antibacterial Article>

The antibacterial article of the disclosure includes the antibacterial metal material of the disclosure.

The antibacterial article is not particularly limited, and can be widely used in various applications, including: medical/pharmaceutical-related applications (e.g., medical pads, surgical instruments, medicine bottle lids, and dental materials); housing-related applications (e.g., door knobs and handrails); food/cooking-related applications (e.g., tableware, cooking utensils, sinks, faucets, and serving trays); infrastructure-related applications (e.g., water treatment and pipes used in plant facilities); automotive-related applications (e.g., door knobs); miscellaneous goods-related applications (e.g., pen cases, rulers, mechanical pencils, and calculators); IT-related applications (e.g., housings of personal computers, smartphones, and the like); and entertainment-related applications (e.g., medals and the like used in gaming machines).

A method of producing the antibacterial article is not particularly limited, and the antibacterial article may be produced by any conventional method using the antibacterial metal material of the disclosure.

### EXAMPLES

The disclosure will now be described more concretely by way of Examples. However, the disclosure is not limited to the below-described Examples.

### <Example 1>

A metal material used for the production of an antibacterial metal material for antibacterial test and a metal material used for the production of an antibacterial metal material for virus inactivation test were prepared as follows.

### (Antibacterial Test)

A metal material was prepared by cutting an aluminum alloy (thickness: 0.2 cm) of the alloy No. A5052 prescribed in JIS H4000:2014 into a size of 4.5 cm in length and 2.0 cm in width. This metal material (sample) had a geometric surface area of 20.6 cm², a density of 2.68 g/cm³, a volume of 1.8 cm³, and a mass of 4.8 g.

### (Virus Inactivation Test)

A metal material was prepared by cutting an aluminum alloy (thickness: 0.2 cm) of the alloy No. A5052 prescribed in JIS H4000:2014 into a size of 3.5 cm in length and 2.4 cm in width. This metal material (sample) had a geometric surface area of 19.2 cm², a density of 2.68 g/cm³, a volume of 1.7 cm³, and a mass of 4.5 g.

### (Degreasing Treatment)

The thus obtained metal materials were degreased under the following conditions.
- Composition: 5% by mass of an aluminum cleaner "NE-6" manufactured by Meltex Inc. and 95% by mass of water
- Temperature: 60°C
- Immersion time: 5 minutes

### (Roughening Treatment)

The thus degreased metal materials were immersed in each of an aqueous hydrochloric acid solution, an aqueous sodium hydroxide solution, and an aqueous nitric acid solution under the following conditions, and then washed with water to obtain a first fine structure.

### <Aqueous Hydrochloric Acid Solution>

- Composition: 1% by mass of hydrochloric acid and 99% by mass of water
- Temperature: 40°C
- Immersion time: 1 minute

### <Aqueous Sodium Hydroxide Solution>

- Composition: 1.5% by mass of sodium hydroxide and 98.5% by mass of water
- Temperature: 40°C
- Immersion time: 4 minutes

### <Aqueous Nitric Acid Solution>

- Composition: 3% by mass of nitric acid and 97% by mass of water
- Temperature: 40°C
- Immersion time: 3 minutes

Next, the metal materials having the first fine structure formed thereon were each immersed in a hydrazine hydrate-containing aqueous solution A and a hydrazine hydrate-containing aqueous solution B under the following conditions, washed with water, and then dried at 70°C or lower, whereby an antibacterial metal material 1 of Example 1 was produced. It is noted here that the antibacterial metal material for antibacterial test and the antibacterial metal material for virus inactivation test are both referred to as "antibacterial metal material 1" without distinction.

### <Hydrazine Hydrate-Containing Aqueous Solution A>

- Composition: 3.5% by mass of hydrazine hydrate and 96.5% by mass of water
- Temperature: 60°C
- Immersion time: 1 minute

### <Hydrazine Hydrate-Containing Aqueous Solution B>

- Composition: 0.5% by mass of hydrazine hydrate and 99.5% by mass of water
- Temperature: 40°C
- Immersion time: 5 minutes

### (Structural Observation)

A surface of the antibacterial metal material 1 and a cross-section perpendicular to the surface were observed under a scanning electron microscope "REGULUS 8220" manufactured by Hitachi High-Tech Science Corporation. The antibacterial metal material 1 had a porous structure (first fine structure) and a fine uneven structure layer (second fine structure), but did not have a specific structure.

### (Roughness Index)

After the antibacterial metal material 1 was degassed by vacuum heating (100 °C), the adsorption isotherm was measured by a krypton adsorption method under the liquid nitrogen temperature (77 K) using BELSORP-MAX (manufactured by MicrotracBEL Corp.), and the specific surface area was determined by the BET method. The specific surface area was multiplied by the mass of the antibacterial metal material to calculate the true surface area (m²) of the antibacterial metal material 1. This true surface area (m²) was divided by the geometric surface area (m²) to determine the roughness index of the antibacterial metal material 1.

It is noted here that, since the changes in the geometric area and the mass before and after the roughening treatment were very small and the effect thereof on the calculated value of the roughness index was negligible, the geometric surface area and the mass of the metal material were used as those of the antibacterial metal material 1.

### (Arithmetic Average Roughness, Ten-Point Average Roughness, and Average Length of Profile Elements)

The macroscopic surface properties (i.e., the average value of the arithmetic average roughness (Ra), the average value of the ten-point average roughness (Rz), and the average value of the average length of profile elements (RSₘ), of the first fine structure) were measured in accordance with JIS B0601:2001 using a surface roughness analyzer "SURFCOM 1400D" manufactured by Tokyo Seimitsu Co., Ltd.

### (Average Thickness of Second Fine Structure)

The average thickness of the second fine structure was calculated from the cross-sectional profile obtained using a scanning electron microscope (SEM). Specifically, using a scanning electron microscope "REGULUS 8220" manufactured by Hitachi High-Tech Science Corporation, SEM images were taken for randomly selected 10 points on the antibacterial metal material, after which the average thickness in a length of 1 µm was measured for 2 arbitrary spots in each image, and the same measurement was performed for other 9 points. An average value of a total of 20 measured points was defined as the average thickness of the second fine structure.

### <Example 2>

An antibacterial metal material 2 of Example 2 was produced and the structure thereof was observed in the same manner as in Example 1, except that the roughening treatment method was changed as described below. The antibacterial metal material 2 had a dendritic layer (second fine structure) but did not have a porous structure (first fine structure) or a specific structure. For this antibacterial metal material 2, the roughness index, the macroscopic surface properties (i.e., the arithmetic average roughness, the ten-point average roughness, and the average length of profile elements), and the average thickness of the second fine structure (dendritic layer) were determined in the same manner as in Example 1. It is noted here that the antibacterial metal material for antibacterial test and the antibacterial metal material for virus inactivation test are both referred to as "antibacterial metal material 2" without distinction.

### (Roughening Treatment)

The antibacterial metal material 2 of Example 2 was produced by bringing the metal material into contact with a Cu²⁺-containing oxidizing acidic aqueous solution under the following conditions and subsequently washing the metal material with water.

### <Cu²⁺-Containing Oxidizing Acidic Aqueous Solution>

- Composition: 5.03% by mass of copper sulfate, 30% by mass of nitric acid, and 64.97% by mass of water
- Temperature: 40°C
- Immersion time: 5 minutes

### <Example 3>

An antibacterial metal material 3 of Example 3 was produced and the structure thereof was observed in the same manner as in Example 1, except that the roughening treatment method was changed as described below. The antibacterial metal material 3 had a porous structure (first fine structure), a dendritic layer (second fine structure), and a specific structure. For this antibacterial metal material 3, the roughness index, the macroscopic surface properties (i.e., the average value of the arithmetic average roughness, the average value of the ten-point average roughness, and the average length of profile elements), and the average thickness of the second fine structure (dendritic layer) were determined in the same manner as in Example 1. It is noted here that the antibacterial metal material for antibacterial test and the antibacterial metal material for virus inactivation test are both referred to as "antibacterial metal material 3" without distinction. In the specific structure of the antibacterial metal material 3, the opening diameter was about 10 µm, and the maximum inner diameter of the inside of pores was about 15 µm.

### (Roughening Treatment)

The metal material that was degreased in the same manner as in Example 1 was immersed in a zinc ion-containing alkaline aqueous solution containing an alkali hydroxide (MOH) and zinc ion (Zn²⁺) at a mass ratio (MOH/Zn²⁺) of from 1 to 100 to form a zinc-containing coating film on the surface.

Subsequently, the metal material was treated with an acid-based etchant containing an acid and at least one of ferric ion or cupric ion under the following conditions to elute the zinc-containing coating film on the surface of the metal material, and this was followed by washing with water.

Thereafter, the antibacterial metal material 3 of Example 3 was produced by bringing the metal material into contact with a Cu²⁺-containing oxidizing acidic aqueous solution under the following conditions and subsequently washing the metal material with water.

### <Zinc Ion-Containing Alkaline Aqueous Solution>

- Composition: 19% by mass of sodium hydroxide, 3.2% by mass of zinc oxide, and 77.8% by mass of water
- Temperature: 30°C
- Immersion time: 2 minutes

### <Acid Etchant>

- Composition: 4.1% by mass of sulfuric acid, 3.9% by mass of iron (II) chloride, 0.2% by mass of copper (II) chloride, and 91.8% by mass of water
- Temperature: 30°C
- Immersion time: 250 seconds

### <Cu²⁺-Containing Oxidizing Acidic Aqueous Solution>

- Composition: 30% by mass of nitric acid, 6.3% by mass of copper (II) oxide, and 63.7% by mass of water
- Temperature: 40°C
- Immersion time: 5 minutes

### <Example 4>

An antibacterial metal material 4 of Example 4 was produced and the structure thereof was observed in the same manner as in Example 3, except that the Cu²⁺-containing oxidizing acidic aqueous solution was changed to a copper sulfate-containing aqueous solution under the below-described conditions. The antibacterial metal material 4 had a porous structure (first fine structure), a dendritic layer (second fine structure), and a specific structure. For this antibacterial metal material 4, the roughness index, the macroscopic surface properties (i.e., the arithmetic average roughness, the ten-point average roughness, and the average length of profile elements), and the average thickness of the second fine structure (dendritic layer) were determined in the same manner as in Example 1. It is noted here that the antibacterial metal material for antibacterial test and the antibacterial metal material for virus inactivation test are both referred to as "antibacterial metal material 4" without distinction. In the specific structure of the antibacterial metal material 4, the opening diameter was about 10 µm, and the maximum inner diameter of the inside of pores was about 15 µm.

### <Copper Sulfate-Containing Aqueous Solution>

- Composition: 30% by mass of nitric acid, 5.03% by mass of copper sulfate, and 64.97% by mass of water
- Temperature: 40°C
- Immersion time: 5 minutes

### <Example 5>

An aluminum plate serving as a substrate was degreased in the same manner as in Example 1 and, on a surface thereof, a base nickel plating layer having a thickness of from about 3 µm to 4 µm was formed.

This base nickel plating layer was formed using an aqueous solution containing nickel sulfate (280 g/L), nickel chloride (45 g/L), and boric acid (40 g/L) as a plating solution at a liquid temperature of 50°C, a pH of 4.3, and a cathode current density of 3 A/dm².

Subsequently, a nickel plating layer having a porous structure of from about 1 µm to 5 µm in thickness was formed on the base nickel plating layer.

This nickel plating layer having a porous structure was formed using a plating solution, which was obtained by adding dodecyltrimethylammonium chloride (10 ml/L) as a substance inhibiting the growth of the plating layer to an aqueous solution containing nickel sulfate (280 g/L), nickel chloride (45 g/L), and boric acid (40 g/L), at a liquid temperature of 50°C, a pH of 4.3, and a cathode current density of 3 A/dm².

The aluminum plate on which the nickel plating layer having a porous structure was thus formed was subjected to an etching treatment in which the aluminum plate was immersed in an aqueous solution (40°C) containing 0.6% by mass of nitric acid and 7.5% by mass of phosphoric acid for 3 minutes.

In this manner, an antibacterial metal material 5 of Example 5 was produced, and the structure thereof was observed. The antibacterial metal material 5 had a porous structure (first fine structure), a pinholder-like layer (second fine structure), and a specific structure. For this antibacterial metal material 5, the roughness index, the macroscopic surface properties (i.e., the arithmetic average roughness, the ten-point average roughness, and the average length of profile elements), and the average thickness of the second fine structure (pinholder-like layer) were determined in the same manner as in Example 1. It is noted here that the antibacterial metal material for antibacterial test and the antibacterial metal material for virus inactivation test are both referred to as "antibacterial metal material 5" without distinction. In the specific structure of the antibacterial metal material 5, the opening diameter and the maximum inner diameter of the inside of pores were both about 2 µm, and parts where the opening diameter was smaller than the maximum inner diameter of the inside of pores were confirmed.

### <Comparative Example 1>

The metal material prior to the roughening treatment that was used in Example 1 was degreased in the same manner as in Example 1 and, using this metal material as Comparative Example 1, the roughness index and the macroscopic surface properties (i.e., the arithmetic average roughness, the ten-point average roughness, and the average length of profile elements) were determined in the same manner as in Example 1. When the structure of the antibacterial metal material 4 of Comparative Example 1 was observed in the same manner as in Example 1, neither the first fine structure nor the second fine structure was observed.

### <Antibacterial Test>

An antibacterial test was conducted in accordance with ISO 21702:2019 using each antibacterial metal material sterilized with ethanol.

As test bacteria, a single species of *Staphylococcus aureus* was used, and the test bacteria inoculation concentration was from 2.5 × 10⁵ cells/mL to 10⁶ cells/mL, and the test bacteria culture solution was 1/25 NB.

In this antibacterial test, the viable cell count ratio was measured 30 minutes and 24 hours after the inoculation of the test bacterial solution on the antibacterial metal material 1.

The viable cell count ratio (%) after 30 minutes indicates a ratio of the viable cell count after a lapse of 30 minutes from the time when the antibacterial metal material 1 was inoculated with the test bacterial solution with respect to the viable cell count at the time when the antibacterial metal material 1 was inoculated with the test bacterial solution. The viable cell count ratio (%) after 24 hours indicates a ratio of the viable cell count after a lapse of 24 hours from the time when the antibacterial metal material 1 was inoculated with the test bacterial solution with respect to the viable cell count at the time when the antibacterial metal material 1 was inoculated with the test bacterial solution. A lower viable cell count ratio (%) indicates a greater number of damaged bacteria.

For the antibacterial metal materials of Examples 1, 2, 4, and 5 as well as the antibacterial metal material of Comparative Example 1, the antibacterial test was conducted in the above-described manner. The results thereof are shown in Table 1.

### <Virus Inactivation Test>

A virus inactivation test was conducted in accordance with ISO 21702:2019 using each antibacterial metal material. In this virus inactivation test, the virus inactivation rate and the infectious titer were measured 30 minutes and 24 hours after the inoculation of the test virus solution on the antibacterial metal material. The infectious titer is an index that represents the degree of virus inactivation. Two virus species, influenza A H3N2 (size: from 80 nm to 120 nm, with envelope) and feline calicivirus (size: from 27 nm to 32 nm, no envelope), were used.

For the antibacterial metal materials of Examples 1, 3, 4, and 5 as well as the metal material of Comparative Example 1, the virus inactivation test was conducted in the above-described manner. It is noted here that, with regard to the antibacterial metal material 1 of Example 1, the virus inactivation rate and the infectious titer after 24 hours were not evaluated for influenza A H3N2. The results are shown in Table 2.

The virus inactivation rate after 30 minutes indicates a ratio of the infectious titer after a lapse of 30 minutes from the time when the antibacterial metal material 1 was inoculated with the test virus solution with respect to the infectious titer at the time when the antibacterial metal material 1 was inoculated with the test virus solution. The virus inactivation rate after 24 hours indicates a ratio of the infectious titer after a lapse of 24 hours from the time when the antibacterial metal material 1 was inoculated with the test virus solution with respect to the infectious titer at the time when the antibacterial metal material 1 was inoculated with the test virus solution. A higher virus inactivation rate indicates that the activities of a greater number of viruses were weakened.

The virus infectious titer was measured in terms of TCID50 (50% tissue culture infectious dose).

**[Table 1]**

| | Roughness index | Macroscopic surface properties [µm] | | | Thickness of second fine structure [µm] | Viable cell count ratio [%] | |
|---|---|---|---|---|---|---|---|
| | | Ra | Rz | RSm | - | after 30 minutes | after 24 hours |
| Example 1 | 14.8 | 0.36 | 2.56 | 91.87 | 0.04 | 61 | 0.202 |
| Example 2 | 12.5 | 0.39 | 2.77 | 99.55 | 0.25 | 21 | 0.054 |
| Example 4 | 102.4 | 4.68 | 24.72 | 108.38 | 0.49 | 2 | 0.016 |
| Example 5 | 14.3 | 0.47 | 2.52 | 150.11 | < 0.20 | 65 | 0.018 |
| Comparative Example 1 | 1.0 | 0.35 | 2.47 | 95.17 | - | 92 | 17.292 |

**[Table 2]**

| | Roughness index | Macroscopic surface properties [µm] | | | Thickness of second fine structure [µm] | Influenza A H3N2 | | | | Feline calicivirus | | | |
|---|---|---|---|---|---|---|---|---|---|---|---|---|---|
| | | | | | | Virus inactivation rate [%] | | Infectious titer [LogTCID50/mL] | | Virus inactivation rate [%] | | Infectious titer [LogTCID50/mL] | |
| | | Ra | Rz | RSm | - | after 30 minutes | after 24 hours | after 30 minutes | after 24 hours | after 30 minutes | after 24 hours | after 30 minutes | after 24 hours |
| Example 1 | 14.8 | 0.36 | 2.56 | 91.87 | 0.04 | 75.5 | 99.023 | 6.15 | 4.75 | 0 | ≥ 99.999 | 7.11 | ≤ 2.28 |
| Example 3 | 55.4 | 5.17 | 29.72 | 112.27 | 0.49 | 98.2 | ≥ 99.997 | 5.01 | ≤ 2.28 | 83.8 | ≥ 99.999 | 6.32 | ≤ 2.28 |
| Example 4 | 102.4 | 4.68 | 24.72 | 108.38 | 0.49 | 97.8 | ≥ 99.997 | 5.10 | ≤ 2.28 | 80.0 | ≥ 99.999 | 6.41 | ≤ 2.28 |
| Example 5 | 14.3 | 0.47 | 2.52 | 150.11 | < 0.20 | 83.8 | 99.999 | 6.02 | 1.85 | 0 | ≥ 99.999 | 7.07 | 0.88 |
| Comparative Example 1 | 1.0 | 0.35 | 2.47 | 95.17 | - | 47.4 | 99.825 | 6.48 | 3.96 | 0 | 99.991 | 7.11 | 3.09 |

The roughness index was less than 4.0 in Comparative Example 1. Therefore, the viable cell count ratio of the antibacterial metal material of Comparative Example 1 after 24 hours was 17%. The virus inactivation rate of the antibacterial metal material of Comparative Example 1 against feline calicivirus after 24 hours was 99.991%.

On the other hand, the roughness index was 4.0 or more in Examples 1 to 5. Therefore, the viable cell count ratio of the antibacterial metal materials of Examples 1, 2, 4, and 5 was 0.202% or lower after 24 hours, which was lower than that of Comparative Example 1. In other words, the antibacterial metal materials of Examples 1, 2, 4, and 5 were found to exhibit antibacterial performance.

The antibacterial metal materials of Examples 1 and 3 to 5 had a virus inactivation rate of 99.999% or higher against feline calicivirus after 24 hours, which was higher than that of Comparative Example 1. In other words, the antibacterial metal materials of Examples 1 and 3 to 5 were found to exhibit virus inactivation performance.

From these results, it was found that the antibacterial metal materials of Examples 1 to 5 had at least one of antibacterial performance or virus inactivation performance. In other words, the antibacterial metal materials of Examples 1 to 5 were found to have excellent antibacterial property.

As shown in Table 1, the antibacterial metal materials of Examples 1, 2, and 4 had excellent antibacterial property with a low viable cell count ratio. Particularly, after 24 hours from the inoculation of the test bacterial solution, the viable cell count ratio was markedly reduced to 0.202% or lower. On the other hand, the metal material of Comparative Example 1 had poor antibacterial property with a high viable cell count ratio.

As shown in Table 2, the antibacterial metal materials of Examples 1, 3, and 4 had a high virus inactivation rate and a low infectious titer, and exhibited excellent antibacterial property. Particularly, after 24 hours from the inoculation of the test virus solution, with regard to feline calicivirus, the virus inactivation rate was markedly increased to 99.999% or higher and the infectious titer was markedly reduced to 2.28 LogTCID50/mL or less. On the other hand, the metal material of Comparative Example 1 had a low virus inactivation rate for influenza A H3N2 after 30 minutes, and exhibited poor antibacterial property.

Further, among the antibacterial metal materials of Examples 1, 3, and 4 that had a first fine structure and a second fine structure layer, the antibacterial metal materials of Examples 3 and 4, which had larger values of Ra, Rz, and RSₘ and a greater thickness of the second fine structure, exhibited superior antibacterial property with a higher inactivation rate even 30 minutes after the inoculation of the test virus solution, as compared to the antibacterial metal material of Example 1. For influenza A H3N2 as well, after 24 hours from the inoculation of the test virus solution, the antibacterial metal materials of Examples 3 and 4 were able to not only markedly increase the virus inactivation rate to 99.997% or higher but also markedly reduce the infectious titer to 2.28 LogTCID50/mL or less.

The disclosure of Japanese Patent Application No. 2021-129074 filed on August 5, 2021 is hereby incorporated by reference in its entirety.

All the documents, patent applications, and technical standards that are described in the present specification are hereby incorporated by reference to the same extent as if each individual document, patent application, or technical standard is concretely and individually described to be incorporated by reference.

## Claims

1. An antibacterial metal material, comprising a surface having a roughness index of 4.0 or more as determined by dividing a true surface area (m²), which is measured by a krypton adsorption method, by a geometric surface area (m²).

2. The antibacterial metal material according to claim 1, wherein the surface has a porous structure.

3. The antibacterial metal material according to claim 2, comprising a plating layer, wherein the surface is a surface of the plating layer.

4. The antibacterial metal material according to claim 2 or 3, comprising a structure in which at least some respective pores among a plurality of pores have an opening diameter that is smaller than a maximum inner diameter of an interior of the respective pores.

5. The antibacterial metal material according to any one of claims 2 to 4, wherein the surface satisfies at least one of the following (1) to (3):
(1) an average value of an arithmetic average roughness (Ra) is from 0.2 µm to 20 µm;
(2) an average value of a ten-point average roughness (Rz) is from 2 µm to 100 µm; and
(3) an average value of an average length (RSₘ) of profile elements is from 10 µm to 400 µm.

6. The antibacterial metal material according to claim 5, wherein:
the average value of the arithmetic average roughness (Ra) is 3 µm or more,
the average value of the ten-point average roughness (Rz) is 20 µm or more, and
the average value of the average length (RSₘ) of profile elements is 100 µm or more.

7. The antibacterial metal material according to any one of claims 1 to 6, wherein the surface comprises an uneven structure layer having an average thickness of from 10 nm to 5,000 nm.

8. The antibacterial metal material according to claim 7, wherein the uneven structure layer is a dendritic layer or a mesh-like layer.

9. The antibacterial metal material according to claim 7 or 8, wherein the average thickness is more than 200 nm.

10. The antibacterial metal material according to claim 7 or 8, wherein the average thickness is 350 nm or more.

11. The antibacterial metal material according to any one of claims 1 to 10, comprising at least one selected from the group consisting of aluminum, magnesium, silver, copper, iron, titanium, an alloy, and a plating material,
wherein the alloy comprises at least one selected from the group consisting of aluminum, magnesium, silver, copper, iron, and titanium.

12. The antibacterial metal material according to any one of claims 1 to 11, wherein the roughness index is 95.0 or more.

13. An antibacterial article, comprising the antibacterial metal material according to any one of claims 1 to 12.
